# FASCICULE DE BREVET EUROPEEN

(11) **EP 0 758 400 B1**
(45) Date de publication et mention de la délivrance du brevet: **17.11.1999**
(21) Numéro de dépôt: 95919470.5
(22) Date de dépôt: 03.05.1995
(51) Int. Cl.: C12Q 1/06, C12M 1/34

(54) **PROCEDE ET INSTALLATION POUR LA NUMERATION DE CELLULES ET MICRO-ORGANISMES, NOTAMMENT DES PRODUITS ALIMENTAIRES OU DES FLUIDES BIOLOGIQUES**
VERFAHREN UND EINRICHTUNG ZUR ZÄHLUNG VON ZELLEN UND MIKROORGANISMEN, IM BESONDEREN IN LEBENSMITTELN UND BIOLOGISCHEN FLÜSSIGKEITEN
METHOD AND APPARATUS FOR COUNTING CELLS AND MICROORGANISMS, PARTICULARLY IN FOOD AND BIOLOGICAL FLUIDS

(30) Priorité: 05.05.1994 FR 9405551
(43) Date de publication de la demande: 19.02.1997
(73) Titulaire: BIOCOM S.A., 91942 Les Ulis Cédex (FR)
(72) Inventeur: BISCONTE, Jean-Claude, F-91640 Briis-sous-Forges (FR)
(74) Mandataire: Pernez, Helga
(86) Numéro de dépôt international: FR9500575
(87) Numéro de publication internationale: WO9530768

(56) Documents cités:
- EP-A- 0 148 290
- EP-A- 0 333 560
- EP-A- 0 397 583
- EP-A- 0 405 480
- EP-A- 0 443 700
- EP-A- 0 465 987
- EP-A- 0 529 084
- WO-A-90/03440

## Description

La présente invention a pour objet un procédé et une installation pour la numération de cellules et micro-organismes, notamment des produits alimentaires ou des fluides biologiques.

On connaît dans l'état de la technique différents procédés pour déterminer la présence de micro-organismes et cellules, notamment des cellules, des levures, moisissures, parasites ou virus dans un produit alimentaire, et de mesurer la concentration desdits micro-organismes et cellules à des fins de contrôle de qualité par exemple.

Un premier procédé consiste à mettre en culture un échantillon du produit à tester, et de procéder après quelques jours d'incubation, a une observation microscopique de la boîte de culture afin de procéder à un comptage des micro-organismes et cellules.

Cette méthode de numération nécessite un délai entre la prise d'échantillon et l'obtention du résultat, et n'est donc pas adaptée à un contrôle en temps réel. De plus, ce procédé aboutit à une évaluation plus qu'à une numération précise.

On a également proposé dans l'état de la technique de marquer les micro-organismes et cellules au moyen de colorants chimiques afin de les rendre fluorescents, rendant ainsi l'identification et le comptage des micro-organismes et cellules plus facile, et automatisable par l'acquisition d'une image microscopique faisant ensuite l'objet d'un traitement du signal par un ordinateur. Ce traitement du signal a pour effet de reconnaître les micro-organismes et cellules marqués en les discriminant par rapport au fond, et à compter le nombre d'objets reconnus.

Afin de rendre la numération plus rapide, on a proposé d'associer ce procédé de marquage et de reconnaissance automatique des objets marqués à un système de filtration augmentant la concentration des micro-organismes et cellules à contrôler.

Le brevet européen EP-A-0 333 560 décrit un tel procédé consistant à mettre tout d'abord l'échantillon en contact avec un anticorps ou une sonde fluorescente, et à compter individuellement les micro-organismes et cellules marquées.

Le système peut également traiter des cellules comme les polynucléaires du lait, ou le sang et les urines humaines. Dans ce cas, le système se substitue à des moyens connus comme les cytomètres de flux.

Ce document de l'art antérieur suggère de procéder au comptage par analyse d'image sous microscope.

La demanderesse a développé une installation de comptage automatique de micro-organismes et cellules et de cellules marqués commercialisée sous le nom commercial de "COBRA", permettant de procéder à un comptage des micro-organismes et cellules en balayant une pluralité de plans focaux d'une membrane, et en procédant pour chaque plan focal à un traitement de l'image afin de reconnaître et de compter les événements recherchés.

Un tel dispositif s'avère très performant pour une analyse en laboratoire et a permis de proposer des tests de routines homologués par les administrations officielles pour le contrôle de produits alimentaires notamment. Les installations de ce type sont toutefois coûteuses car elles mettent en oeuvre des moyens d'acquisition d'image performants et des systèmes de traitement d'image élaborés. Ils sont par ailleurs difficilement compatibles avec une utilisation en dehors d'un laboratoire, sur le terrain et peu accessibles à des petits laboratoires.

Il présente de plus deux inconvénients importants. Premièrement, le comptage selon l'état de la technique s'effectue par échantillonnage de la surface totale de la membrane, chaque image correspondant à un champ d'observation particulier parmi plusieurs centaines ou milliers de champs d'observation possible. Pour que le comptage présente un taux d'erreur acceptable, il convient de multiplier les champs d'observation à l'intérieur desquels on procède à un comptage automatique, le nombre de champ à analyser étant déterminé suivant les lois de la statistiques, et en fonction du temps que l'on peut raisonnablement affecter à une analyser. Dans le cas où le nombre de micro-organisme et cellules, et donc la densité d'événements, est très faible, le taux d'erreur devient important. De plus, la répartition des échantillons n'est pas homogène, et il existe notamment des effets de bords.

On a dans l'état de la technique tenté de réduire ce taux d'erreurs en procédant à un balayage de la membrane en faisant varier la position du champ d'observation et de la profondeur du champ d'observation. Cela entraîne une multiplication des séquences d'analyse d'image, et donc un temps d'analyse accrue.

Un deuxième inconvénient concerne les situations où la densité d'événement est élevée, et entraîne l'agglomération de micro-organisme et cellules. Les algorithmes de comptage automatique peuvent dans ce cas assimiler une agglomération de plusieurs centaines de micro-organismes et cellules à un événement unique, et donc fournir un résultat grossièrement erroné.

L'évolution des appareillages automatiques montre que la tendance naturelle de l'homme de métier est de perfectionner continuellement le comptage individuel des événements, en passant du comptage visuel de colonies formées par une culture sur un milieu nutritif dans une boîte de PETRI, à l'association de la filtration sur membrane et du comptage automatique par traitement d'image, puis à l'automatisation du déplacement du microscope en position X-Y et en profondeur de champ pour multiplier le nombre de champs analysés. Une autre approche, également sur un principe de comptage individuel d'événements et connue sous le nom de cytométrie de flux, consiste à compter individuellement le passage d'un événement dans un flux porteur.

La présente invention va radicalement à l'encontre de cette démarche qui consiste à améliorer les techniques de comptages unitaire des événements, en proposant une évaluation du nombre d'événements par une acquisition globale de l'intensité lumineuse des micro-organismes et cellules marqués fixés sur un support.

Le problème que l'invention vise à résoudre est d'augmenter la fiabilité de la détermination du nombre de cellules ou micro-organismes, notamment dans les cas de très faibles concentrations ou de très fortes concentrations.

L'objet de la présente invention est de proposer un procédé permettant de procéder à une analyse rapide de fluides biologiques à l'aide d'une installation simple et robuste, dont le prix de revient est sensiblement inférieur aux dispositifs de l'état de la technique, et dont le fonctionnement est compatible avec une utilisation sur le terrain. En particulier, la mobilité de l'installation est souhaitable pour procéder au contrôle rapide de lait cru au niveau d'un centre de production, en vue de la recherche individuelle de la source d'infection en cas de présence de cellules somatiques dépassant les normes admises, et pour le contrôle immédiat à des fins de "paiement à la qualité" ou de contrôle sanitaire des animaux producteurs.

Afin de répondre à ces objectifs, l'invention concerne plus particulièrement un procédé de numération de cellules et micro-organismes et cellules présents dans un milieu fluide, notamment des produits alimentaires ou des fluides biologiques, consistant à procéder à un marquage avec un marqueur coloré ou fluorescent marquant sélectivement les micro-organismes et cellules à détecter et à procéder au comptage des micro-organismes et cellules ainsi marqués, caractérisé en ce que le marqueur coloré ou fluorescent est un marqueur stoechiométrique, notamment un marqueur du DNA ou un marqueur du RNA, en ce que l'on procède, simultanément ou préalablement au comptage, à une concentration des micro-organismes et cellules par une étape de filtration du milieu fluide, le comptage étant réalisé par la mesure de l'intensité lumineuse globale du milieu concentré et par comparaison de ladite intensité lumineuse avec une courbe de calibration.

Le procédé selon l'invention permet de procéder avec une précision suffisante et une reproductibilité améliorée pour la plupart des contrôles de routine à une détermination de la concentration cellulaire dans un fluide biologique, sans recours à des techniques complexes de traitement d'image, et fournissant un résultat dont la fiabilité n'est pas dégradée en cas de très faible ou de très forte concentration, contrairement aux procédés de comptage individuels de l'état de la technique.

En particulier, pour le contrôle de lait, le procédé selon l'invention permet de déterminer des concentrations variant entre 50.000 cellules et 1.000.000 cellules par millilitre de lait, dans un délai de l'ordre de la seconde pour un à 24 échantillons simultanés.

Selon une première variante, le marqueur est un colorant marquant sélectivement les micro-organismes et cellules à détecter, et en ce que le comptage est réalisé par la mesure de l'intensité lumineuse globale du milieu éclairé et par comparaison de ladite intensité lumineuse avec une courbe de calibration. La mesure s'effectue par mesure globale de la densité optique des événements marqués.

Selon une deuxième variante, le marqueur est un marqueur fluorescent marquant sélectivement les micro-organismes et cellules à détecter constitué par une sonde fluorescente, et en ce que le comptage est réalisé par la mesure de l'intensité lumineuse globale du milieu concentré dans la bande de longueur d'onde de réémission de l'un au moins des marqueurs fluorescents et par comparaison de ladite intensité lumineuse avec une courbe de calibration.

La concentration peut être réalisée de différentes manière, par utilisation de micro-billes magnétiques portant des anticorps spécifiques, par fixation sur une surface microporeuse ou non portant des anticorps greffés, ou de préférence par filtration sur une membrane microporeuse.

Selon un mode de mise en oeuvre préféré, le procédé de numération comporte une étape de concentration des micro-organismes et cellules par une étape de filtration mettant en oeuvre une membrane microporeuse transparente dans les longueurs d'onde d'excitation des marqueurs fluorescents dont la surface est normalisée, le comptage étant réalisé par la mesure de l'intensité lumineuse globale de la membrane de filtration de surface normalisée dans la bande de longueur d'onde de réémission, de l'un au moins des marqueurs fluorescents et par comparaison de ladite intensité lumineuse avec une courbe de calibration.

Avantageusement, on procède à la mesure de l'intensité lumineuse par intégration du signal provenant d'un capteur photovoltaïque pendant un interval de temps prédéterminé.

Selon une autre variante, on mesure l'intensité lumineuse dans plusieurs bandes de longueur d'onde différente correspondant aux bandes de réémission de marqueurs différents. Cette variante permet de détecter la présence de différents types cellulaires.

L'invention concerne également une installation pour la numération de cellules et micro-organismes et cellules du type comportant un support de membrane microporeuse, une source de lumière pour l'éclairage de la membrane et des moyens d'analyse des filtrats déposés sur la membrane, les moyens d'analyse étant constitués par au moins un capteur photovoltaïque propre à détecter l'intensité lumineuse globale émise par un élément de surface déterminé de la membrane.

Avantageusement, l'invention comporte un tiroir pour le positionnement d'une membrane microporeuse, une source de lumière émettant dans la plage de longueur d'onde d'excitation des marqueurs fluorescents disposée d'un coté dudit tiroir, et au moins un capteur muni d'un filtre dont la bande passante correspond sensiblement à la bande de réémission du marqueur fluorescent, ledit capteur et ledit filtre étant disposés du coté opposé au tiroir.

L'invention peut également fonctionner en mesure de densité par transmission ou réflexion dans le domaine du visible.

Selon une première variante de réalisation, l'installation comporte un capteur CCD propre à détecter l'intensité lumineuse d'un élément surfacique de membrane microporeuse.

Selon une variante de réalisation préférée, l'installation comporte une pluralité de photodiodes propre à détecter l'intensité lumineuse d'un élément surfacique de membrane microporeuse.

Avantageusement, les photodiodes sont logées dans des alésages cylindriques dont la répartition correspond aux zones de filtration de la membrane microporeuse, le tiroir comportant des moyens d'indexation pour le positionnement de la membrane par rapport audits alésages.

L'invention sera mieux comprise dans ce qui suit, à la lecture de la description faisant référence aux dessins annexés où :
- la figure 1 représente une vue en coupe d'un exemple de réalisation d'une installation selon l'invention ;
- la figure 2 représente une vue de dessus de la membrane microporeuse placée dans son support semi-rigide ;
- la figure 3 représente un schéma de principe des circuits électroniques mis en oeuvre par l'installation.

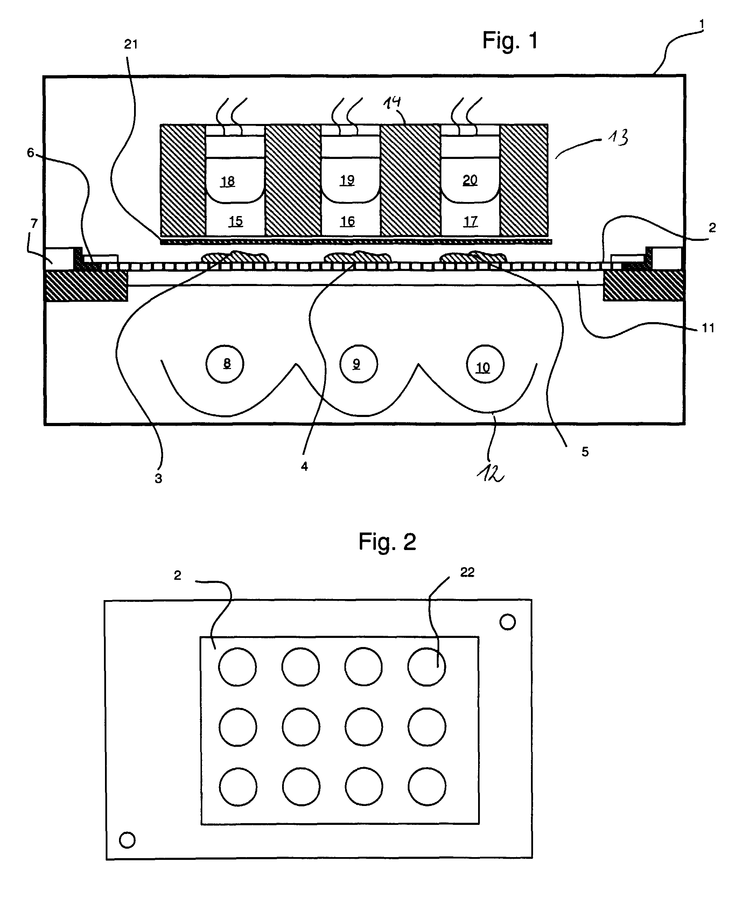

La figure 1 représente une vue schématique, en coupe, d'un exemple de réalisation d'une installation de numération de cellules selon l'invention.

L'installation comporte un boîtier (1) rigide s'ouvrant par une trappe permettant l'introduction d'une membrane microporeuse (2) dans un support souple indexé portant des filtrats (3 à 5). Cette membrane (2) est placée sur un support métallique formant un tiroir coulissant entre deux rails de guidages (6, 7).

Une rampe de sources ultraviolet (8 à 10) est disposée en dessous du tiroir portant la membrane (2). Un filtre passe-bande (11) laissant passer les rayonnements U.V. dans la longueur d'onde d'excitation des marqueurs est intercalé entre la rampe de sources U.V. (8 à 10) et la membrane (2). Un réflecteur métallisé (12) renvoie une partie du rayonnement vers le filtre (11). Du coté opposé à la rampe U.V., l'installation comporte un ensemble de photodétecteurs (13) constitué par un bloc (14) présentant une pluralité d'alésages (15, 16, 17) dirigés en direction de la membrane (2). Dans chacun de ces alésages (15 à 17) de forme cylindrique est logée une photodiode (18 à 20). Ces photodiodes (18 à 20) sont organisés en matrice dont la géométrie correspond à la géométrie de l'installation de filtration assurant le dépôt de filtrats (3 à 5) sur la membrane (2).

Un filtre (21) est interposé entre la membrane (2) et le bloc de photodétecteurs (14). Ce filtre est un passe-bande laissant passer les longueurs d'onde de réémission des marqueurs fluorescents.

La figure 2 représente une vue de dessus d'un exemple de membrane microporeuse posée intégrée dans son support souple de type COBRA (Marque déposée par la demanderesse).

La membrane (2) présente une matrice de zones de filtration (22) dont la configuration est déterminée par l'appareil de filtration employé, et qui correspond à la disposition des photodiodes.

Le fonctionnement de l'installation de numération selon l'invention sera exposé dans ce qui suit, en référence à un exemple d'application concerne le contrôle rapide de lait cru.

On prélève un échantillon de lait cru et on procède à un éventuel prétraitement pour digérer les constituants indésirables tels que les globules gras et l'addition d'un colorant fluoréscent.

L'échantillon de lait est ensuite filtré sur une membrane en polycarbonate ou en Polyéthylène téréphtalate présentant des pores d'une section de 2 microns sur une installation de filtration telle que le matériel "COBRA" de la société BIOCOM. L'installation de filtration permet la filtration simultanée de plusieurs échantillons, et le dépôt d'une pluralité de filtrats sur la membrane, en forme de matrice. La sélectivité du marquage n'exige pas de procéder à un rinçage.

Le colorant mis en oeuvre pour le marquage est par exemple du bromure d'éthidium.

Après séchage à l'air, la membrane est ensuite placée dans l'installation de numération comportant une source d'excitation UV à 360 nm et des photodiodes associées à un filtre laissant passer les rayonnements dans la bande de longueurs d'onde de l'ordre de 600 nanomètres par exemple.

La courbe de calibration est obtenue par comparaison du résultat obtenu par l'installation selon l'invention, et le résultat obtenu ultérieurement, par exemple en laboratoire, avec des procédés connus dans l'art antérieur.

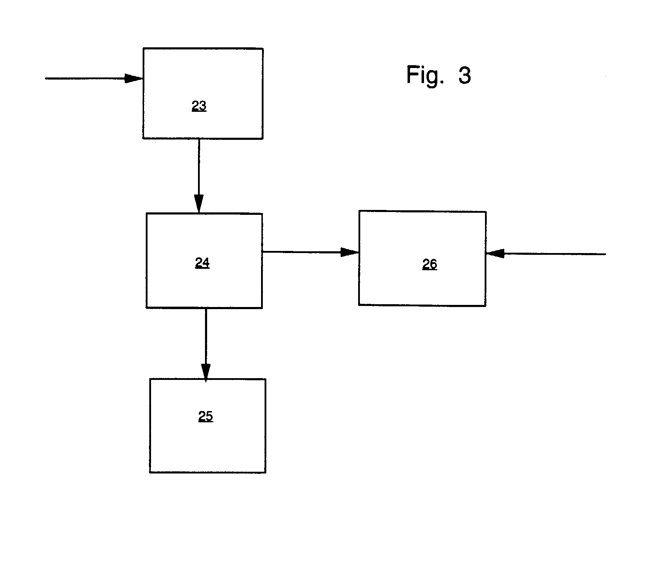

La figure 3 représente un schéma de principe des circuits électroniques mis en oeuvre par l'installation.

Le signal provenant de chacune des photodiodes est numérisé par un circuit de digitalisation (23) relié à un microprocesseur (24) assurant le traitement du signal et le calcul du nombre de cellules en fonction de la courbe de calibration et de l'intensité lumineuse mesurée par la photodiode. Le résultat est affiché sur un afficheur digital (25) et est enregistré dans une mémoire vive (26). Cette mémoire contient un fichier constitué du numéro de l'enregistrement, de la valeur du signal provenant de la photodiode, et du nombre de cellules détectées.

Cette mémoire peut par ailleurs recevoir les résultats de la numération, selon un procédé connu, du même échantillon. Le calculateur (24) recalcule en fonction de ces données une nouvelle courbe de calibration.

La numération de cellules ou de micro-organismes selon l'invention s'effectue selon un protocole dont un exemple est indiqué ci-après, pour la numération du nombre de cellules dans le lait :
- on prélève un échantillon de 30 µl de lait ;
- on dilue cet échantillon dans 1 ml d'eau distillée ;
- on ajoute 0,5 % de formaldéhyde agissant comme fixateur et 5 µg de bromure d'éthidium par ml de solution à tampon PH 7 ;
- on chauffe l'échantillon à 60 degrés centigrades pendant 20 minutes ;
- on procède à la filtration de l'échantillon prétraité sur une membrane en polycarbonate présentant une porosité de 2 microns, sous une dépression de 0,5 bars ou une pression de 0,8 bars
- on place la membrane sur le dispositif conforme à l'invention pour mesurer la luminosité globale, par excitation par une source émettant à 360 nm et observation dans le rouge, à 580 nm.

Un exemple de protocole pour la préparation d'échantillons pour la numération des bactéries dans le lait consiste à :
- prélever 100 µl de lait ;
- procéder à la dilution de cet échantillon dans 800 µl d'un mélange comprenant les produits suivants :
- trypsine à 1,33 mg/ml
- triton à 0,5 %

Ce mélange provoque la digestion du lait par éclatement des cellules somatiques et des protéines du lait. Les bactéries, préservées par leur paroi résistante à cette digestion, ne sont pas détruites.
- chauffage pendant 10 minutes à 47 °C ;
- filtration sur une membrane de porosité 0,6 µm ;
- Rinçage tampon nitrate PH 3
- coloration à l'acridine 0,025 PH 4 pendant 3 minutes ;
- rinçage avec un tampon CITRATE PH 3 ;
- lecture avec le dispositif selon l'invention, par excitation par une source émettant à 260 nm et observation dans l'orangé, à 580 nm.

Un autre exemple de protocole pour la préparation d'échantillons pour le phénotypage des leucémies consiste à
- fixer par greffage sur la surface de la membrane des anticorps reconnaissant des lymphocytes déterminés un échantillon de 5 ml de sang ;
- procéder à l'immersion et à l'incubation pendant 20 minutes, avec agitation douce, du récipient pour favoriser l'adhésion des lymphocytes ;
- procéder au rinçage de la membrane dans un tampon ;
- procéder à la coloration dans du bromure d'éthilium ;
- procéder à la lecture des différentes zones de fixations des lymphocytes pour obtenir un profil de la population.

Un autre exemple de protocole pour la préparation d'échantillons pour le phénotypage de leucémies consiste à :
- fixer des anticorps sur des billes magnétiques d'un diamètre de 1 micron ;
- laisser incuber pendant 15 minutes dans 1 ml de sang ;
- introduire un aimant pour retenir le complexe billes/cellules ;
- éliminer les résidus ;
- procéder à la filtration des complexes retenus par l'aimant sur une membrane permettant le passage des billes libres, mais pas des cellules ;
- procéder à une coloration au bromure d'éthidium;
- procéder à la numération par mesure globale conformément à l'invention.

## Revendications

1. Procédé de numération de cellules et micro-organismes et cellules présents en très faible concentration telle que 50000 à 1 000 000 de cellules par millilitre dans un milieu fluide, notamment des produits alimentaires ou des fluides biologiques, consistant à procéder à un marquage avec un marqueur coloré ou fluorescent marquant sélectivement les micro-organismes et cellules à détecter et à procéder au comptage des micro-organismes et cellules ainsi marqués, caractérisé en ce que le marqueur coloré ou fluorescent est un marqueur stoechiométrique, notamment un marqueur du DNA ou un marqueur du RNA, en ce que l'on procède, simultanément ou préalablement au comptage, à une concentration des micro-organismes et cellules par une étape de concentration du milieu fluide, le comptage étant réalisé par la mesure de l'intensité lumineuse globale du milieu concentré à l'aide d'un photodétecteur et par comparaison de ladite intensité lumineuse avec une courbe de calibration, cette mesure consistant soit en une mesure de densité par transmission ou réflexion dans le domaine du visible, soit en une mesure de réémission d'un marqueur fluorescent des microorganismes et/ou cellules marqués fixés sur un support.

2. Procédé de numération de cellules e micro-organismes et cellules selon la revendication 1 caractérisé en ce que le marqueur est un colorant marquant sélectivement les micro-organismes et cellules à détecter, et en ce que le comptage est réalisé par la mesure de l'intensité lumineuse globale du milieu éclairé et par comparaison de ladite intensité lumineuse avec une courbe de calibration.

3. Procédé de numération de cellules et micro-organismes et cellules selon la revendication 1 caractérisé en ce que le marqueur est un marqueur fluorescent marquant sélectivement les micro-organismes et cellules à détecter constitué par une sonde fluorescente, et en ce que le comptage est réalisé par la mesure de l'intensité lumineuse globale du milieu concentré dans la bande de longueur d'onde de réémission de l'un au moins des marqueurs fluorescents et par comparaison de ladite intensité lumineuse avec une courbe de calibration.

4. Procédé de numération de cellules et micro-organismes et cellules présents dans un milieu fluide selon la revendication 3, caractérisé en ce que le marqueur fluorescent est un marqueur stoechiométrique, notamment un marqueur du DNA ou un marqueur du RNA constitué par une sonde portant une molécule fluorescente, en ce que l'on procède, simultanément ou préalablement au comptage, à une concentration des micro-organismes et cellules par une étape de filtration mettant en oeuvre une membrane (2) microporeuse transparente dans les longueurs d'onde d'excitation des marqueurs fluorescents dont la surface est normalisée, le comptage étant réalisé par la mesure de l'intensité lumineuse globale de la membrane (2) de filtration de surface normalisée dans la bande de longueur d'onde de réémission de l'un au moins des marqueurs fluorescents et par comparaison de ladite intensité lumineuse avec une courbe de calibration.

5. Procédé de numération de cellules et micro-organismes et cellules selon l'une quelconque des revendications 1 à 4 caractérisé en ce que l'on procède à la mesure de l'intensité lumineuse par intégration du signal provenant d'un capteur photovoltaïque pendant un intervalle de temps prédéterminé.

6. Procédé de numération de cellules et micro-organismes et cellules selon l'une quelconque des revendications 3 à 5 caractérisé en ce que l'on mesure l'intensité lumineuse dans plusieurs bandes de longueurs d'onde différentes correspondant aux bandes de réémission de marqueurs différents.

7. Procédé de numération de cellules et micro-organismes et cellules selon l'une quelconque des revendications précédentes caractérisé en ce que la courbe de calibration est recalculée par l'enregistrement des couples de données constituées par la valeur de l'intensité lumineuse et le résultat d'un comptage par analyse d'image d'un même filtrat.

8. Installation pour la numération de cellules et micro-organismes et cellules du type comportant un moyen de support du milieu fluide concentrer à analyser, une source de lumière pour l'éclairage du moyen de support et des moyens d'analyse, caractérisée en ce que les moyens d'analyse sont constitués par au moins un capteur photovoltaïque propre à détecter l'intensité lumineuse globale émise par un élément de surface déterminé du moyen de support.

9. Installation pour la numération de cellules et micro-organismes et cellules selon la revendication 8 caractérisée en ce qu'elle comporte un support de membrane (2) microporeuse, une source de lumière pour l'éclairage de la membrane (2) et des moyens d'analyse des filtrats (3 à 5) déposés sur la membrane (2), les moyens d'analyse étant constitués par au moins un capteur photovoltaïque propre à détecter l'intensité lumineuse globale émise par un élément de surface déterminé de la membrane (2).

10. Installation pour la numération de cellules et micro-organismes et cellules selon la revendication 9 caractérisé en ce qu'elle comporte un tiroir pour le positionnement d'une membrane (2) microporeuse, une source de lumière émettant dans la plage de longueur d'onde d'excitation des marqueurs fluorescents disposée d'une coté dudit tiroir, et au moins un capteur muni d'un filtre (11) dont la bande passante correspond sensiblement à la bande de réémission du marqueur fluorescent, ledit capteur et ledit filtre étant disposés du coté opposé au tiroir.

11. Installation pour la numération de cellules et micro-organismes et cellules selon l'une quelconque des revendications 9 ou 10 caractérisée en ce qu'elle comporte une pluralité de photodiodes propre à détecter l'intensité lumineuse d'un élément surfacique de membrane (2) microporeuse.

12. Installation pour la numération de cellules et micro-organismes et cellules selon la revendication 10 caractérisée en ce que les photodiodes sont logées dans des alésages cylindriques dont la répartition correspond aux zones de filtration de la membrane (2) microporeuse, le tiroir comportant des moyens d'indexation pour le positionnement de la membrane (2)s par rapport audits alésages.

## Patentansprüche

1. Beschriftungsverfahren von Zellen und Mikro-Organismen und Zellen, die in sehr schwacher Konzentration wie beispielsweise 50000 bis 1 000 000 Zellen pro Milliliter in einem flüßigen Milieu vorhanden sind, insbesondere Nahrungsmittel oder biologische Medien, das darin besteht, die zu detektierenden Mikro-Organismen und Zellen selektiv mit einem farbigen oder fluoreszierenden Markierer zu markieren und die markierten Mikro-Organismen und Zellen zu zählen, dadurch gekennzeichnet, daß der farbige oder fluoreszierende Markierer ein stöchiometrischer Markierer, insbesondere ein DNA- oder ein RNA-Markierer ist, daß man gleichzeitig oder vor dem Zählen die Mikro-Organismen und Zellen durch eine Konzentrationsetappe des flüßigen Milieus konzentriert, wobei das Zählen durch die Messung der Gesamtleuchtkraft des konzentrierten Milieus mittels eines Photozellendetektors und durch Vergleich der besagten Leuchtkraft mit einer Kalibrierkurve erfolgt, wobei diese Messung entweder aus einer Dichtemessung durch Übertragung oder Abstrahlung im sichtbaren Bereich oder aus einer Wiederausstrahlungsmessung eines fluoreszierenden Markierers der markierten, auf einem Träger fixierten Mikro-Organismen und/oder Zellen besteht.

2. Beschriftungsverfahren von Zellen und Mikro-Organismen und Zellen nach Anspruch 1, dadurch gekennzeichnet, daß der Markierer ein Farbstoff ist, der die zu detektierenden Mikro-Organismen und Zellen selektiv markiert, und daß das Zählen durch die Messung der Gesamtleuchtkraft des beleuchteten Milieus und durch Vergleich der besagten Leuchtkraft mit einer Kalibrierkurve erfolgt.

3. Beschriftungsverfahren von Zellen und Mikro-Organismen und Zellen nach Anspruch 1, dadurch gekennzeichnet, daß der Markierer ein fluoreszierender Markierer ist, der die zu detektierenden Mikro-Organismen und Zellen selektiv markiert und aus einer fluoreszierenden Sonde besteht, und daß das Zählen durch die Messung der Gesamtleuchtkraft des konzentrierten Milieus auf dem Wellenlängenband der Wiederausstrahlung von mindestens einem der fluoreszierenden Markierern und durch Vergleich der besagten Leuchtkraft mit einer Kalibrierkurve erfolgt.

4. Beschriftungsverfahren von Zellen und Mikro-Organismen und Zellen in einem flüßigen Milieu nach Anspruch 3, dadurch gekennzeichnet, daß der fluoreszierende Markierer ein stöchiometrischer Markierer, insbesondere ein DNA-oder ein RNA-Markierer ist und aus einer ein fluoreszierendes Molekül tragenden Sonde besteht, daß man gleichzeitig oder vor dem Zählen die Mikro-Organismen und Zellen durch eine Filteretappe konzentriert, wozu man eine mikroporöse, transparente Membran (2) in den Erregungswellenlängen der fluoreszierenden Markierer verwendet, deren Fläche genormt ist, wobei das Zählen durch die Messung der Gesamtleuchtkraft der Filtermembran (2) mit genormter Fläche auf dem Wellenlängenband der Wiederausstrahlung von mindestens einem der fluoreszierenden Markierern und durch Vergleich der besagten Leuchtkraft mit einer Kalibrierkurve erfolgt.

5. Beschriftungsverfahren von Zellen und Mikro-Organismen und Zellen nach einem der Ansprüche 1 bis 4, dadurch gekennzeichnet, daß man die Leuchtkraft durch Integration des von einem photo-voltaischen Geber kommenden Signals während eines vorgegebenen Zeitraumes mißt.

6. Beschriftungsverfahren von Zellen und Mikro-Organismen und Zellen nach einem der Ansprüche 3 bis 5, dadurch gekennzeichnet, daß man die Leuchtkraft auf mehreren verschiedenen Wellenlängenbändern mißt, die den Wiederausstrahlungsbändern von verschiedenen Markierern entsprechen.

7. Beschriftungsverfahren von Zellen und Mikro-Organismen und Zellen nach einem der vorstehenden Ansprüche, dadurch gekennzeichnet, daß die Kalibrierkurve durch die Aufzeichnung der Datenpaare neuberechnet wird, die aus dem Wert der Leuchtkraft und dem Ergebnis einer Zählung durch Bildanalyse eines gleichen Filtrats bestehen.

8. Anlage für die Beschriftung von Zellen und Mikro-Organismen und Zellen des Typs mit einem Trägermittel des zu analysierenden konzentrierten flüßigen Milieus, einer Lichtquelle für die Beleuchtung des Trägermittels und Analysemitteln, dadurch gekennzeichnet, daß die Analysemittel aus mindestens einem photovoltaischen Geber bestehen, der geeignet ist, die Gesamtleuchtkraft zu detektieren, die von einem bestimmten Flächenelement des Trägermittels abgegeben wird.

9. Anlage für die Beschriftung von Zellen und Mikro-Organismen und Zellen nach Anspruch 8, dadurch gekennzeichnet, daß sie einen mikroporösen Membran-Träger (2) umfaßt, eine Lichtquelle für die Beleuchtung der Membran (2) und Analysemittel der auf der Membran (2) abgelagerten Filtrate (3 bis 5), wobei die Analysemittel aus mindestens einem photovoltaischen Geber bestehen, der geeignet ist, die Gesamtleuchtkraft zu detektieren, die von einem bestimmten Flächenelement der Membran (2) abgegeben wird.

10. Anlage zur Beschriftung von Zellen und Mikro-Organismen und Zellen nach Anspruch 9, dadurch gekennzeichnet, daß sie einen Einschub für die Positionierung einer mikroporösen Membran (2) umfaßt, eine Lichtquelle, die auf den Erregungswellen-Längenbereich der fluoreszierenden Markierer leuchtet und auf einer Seite des besagten Einschubs angeordnet ist, und mindestens einen mit einem Filter (11) ausgestatteten Geber, dessen Durchlaßbereich etwa dem Wiederausstrahlungsband des fluoreszierenden Markierers entspricht, wobei der besagte Geber und der besagte Filter auf der dem Einschub gegenüberliegenden Seite angeordnet sind.

11. Anlage zur Beschriftung von Zellen und Mikro-Organismen und Zellen nach einem der Ansprüche 9 oder 10, dadurch gekennzeichnet, daß sie eine Mehrzahl von Photodioden umfaßt, die geeignet sind, die Leuchtkraft eines Flächenelementes der mikroporösen Membran (2) zu detektieren.

12. Anlage zur Beschriftung von Zellen und Mikro-Organismen und Zellen nach Anspruch 10, dadurch gekennzeichnet, daß die Photodioden in zylindrischen Bohrungen angeordnet sind, deren Verteilung den Filterzonen der mikroporösen Membran (2) entspricht, wobei der Einschub Indexierungsmittel für die Positionierung der Membran (2) in Bezug auf die besagten Bohrungen umfaßt.

## Claims

1. A procedure for counting cells and micro-organisms and cells present in very low concentration such as 50,000 to 1,000,000 cells per millilitre in a fluid medium, in particular food products or biological fluids, consisting of marking the micro-organisms and cells to be detected by marking selectively with a coloured or fluorescent marker and proceeding to count the micro-organisms and cells thus marked, characterised in that the coloured or fluorescent marker is a stoechiometric marker, in particular a DNA marker or an RNA marker, in that one proceeds, simultaneously or before the count, by concentrating the micro-organisms and cells through a concentration stage of the fluid medium, the count being carried out by measurement of the global luminous intensity of the concentrated medium using a photodetector and by comparing said luminous intensity with a calibration curve, this measurement consisting either of a density measurement by transmission or reflection in the visible range or a re-emission measurement of a fluorescent marker of the marked micro-organisms and/or cells fixed on a support.

2. A procedure for counting cells and micro-organisms and cells according to Claim 1, characterised in that the marker is a dye selectively marking the micro-organisms and cells to be detected, and in that the count is carried out by measurement of the global luminous intensity of the illuminated medium and by comparison of said luminous intensity with a calibration curve.

3. A procedure for counting cells and micro-organisms and cells according to Claim 1, characterised in that the marker is a fluorescent marker selectively marking the micro-organisms and cells to be detected constituted by a fluorescent sensor, and in that the count is carried out by measurement of the global luminous intensity of the concentrated medium in the re-emission wavelength band of at least one of the fluorescent markers and by comparison of said luminous intensity with a calibration curve.

4. A procedure for counting cells and micro-organisms and cells present in a fluid medium according to Claim 3, characterised in that the fluorescent marker is a stoechiometric marker, in particular a DNA marker or an RNA marker constituted by a sensor carrying a fluorescent molecule, in that one proceeds, simultaneously or before the count, by concentrating the micro-organisms and cells by a filtration stage using a micro-porous membrane (2) transparent in the excitation wavelengths of the fluorescent markers whose surface is normalised, the count being carried out by measurement of the global luminous intensity of the normalised surface filtration membrane (2) in the re-emission wavelength band of at least one of the fluorescent markers and by comparison of said luminous intensity with a calibration curve.

5. A procedure for counting cells and micro-organisms and cells according to any one of Claims 1 to 4, characterised in that one proceeds with the measurement of the luminous intensity by integrating the signal from a photovoltaic sensor over a predetermined time interval.

6. A procedure for counting cells and micro-organisms and cells according to any one of Claims 3 to 5, characterised in that one measures the luminous intensity in several different wavelength bands corresponding to the re-emission bands of different markers.

7. A procedure for counting cells and micro-organisms and cells according to any one of the preceding Claims, characterised in that the calibration curve is recalculated by registering data coupling constituted by the value of the luminous intensity and the result of an image analysis count of a same filtrate.

8. An installation for counting cells and micro-organisms and cells of the type comprising a means of support of the concentrated fluid medium to be analysed, a light source for illuminating the means of support and means of analysis, characterised in that the means of analysis are constituted by at least one photovoltaic sensor capable of detecting the global luminous intensity emitted by a determined surface element of the means of support.

9. An installation for counting cells and micro-organisms and cells according to Claim 8, characterised in that it comprises a micro-porous membrane support (2), a light source for illuminating the membrane (2) and means for analysing the filtrates (3 to 5) deposited on the membrane (2), the means of analysis being constituted by at least one photovoltaic sensor capable of detecting the global luminous intensity emitted by a determined surface element of the membrane (2).

10. An installation for counting cells and micro-organisms and cells according to Claim 9, characterised in that it comprises a slide for positioning a micro-porous membrane (2), a light source emitting in the excitation wavelength range of the fluorescent markers positioned on one side of said slide, and at least one sensor provided with a filter (11) whose passband corresponds closely to the re-emission band of the fluorescent marker, said sensor and said filter being positioned on the opposite side of the slide.

11. An installation for counting cells and micro-organisms and cells according to either of the Claims 9 or 10, characterised in that it comprises a plurality of photodiodes capable of detecting the luminous intensity of a surface element of the micro-porous membrane (2).

12. An installation for counting cells and micro-organisms and cells according to Claim 10, characterised in that the photodiodes are housed in cylindrical bores whose distribution corresponds to the filtration zones of the micro-porous membrane (2), the slide comprising indexing means for positioning the membrane (2) relative to said bores.
